# EUROPEAN PATENT APPLICATION

(11) **EP 4 644 894 A1**
(43) Date of publication of application: **05.11.2025**
(21) Application number: 25172032.2
(22) Date of filing: 23.04.2025
(51) Int. Cl.: G01N 33/00, A01G 7/02

(54) **GAS EXCHANGE MEASUREMENT DEVICE IN A PLANT**

(30) Priority: 30.04.2024 IT 202400009811
(71) Applicant: Plant Flow S.r.l., 10135 Torino (IT)
(72) Inventor: Patono, Davide Lucien, 10135 Torino (IT)
(74) Representative: Mola, Edoardo

(57) **Abstract**

Device for measuring gas exchange in a plant comprising a container, a closing element releasably engaged on the container so as to fluidically isolate such container from the external environment, a first and a second hollow body housed in the container and rigidly connected to each other in a releasable manner defining a first chamber, and the space in the container around the first and the second hollow body defining a second chamber, and said first and second body being further shaped so as to have respectively a first and a second recessed portion in use facing defining a cavity, and the first chamber being accessible from the second chamber via such cavity, and one of the first and second chambers being fluidically connectable at the inlet and outlet respectively to a delivery line and a gas evacuation line to define a controlled atmosphere in the device when the closing element is in operation, a fluidic seal interposed between the first and the second hollow body, an elastically deformable polymeric element releasably engaged in the cavity, and comprising a longitudinal hole defining a passage for the stem of a plant having roots in the first chamber and the crown in the second chamber, a pusher engaged in the cavity on the side of the second chamber in a movable manner to the translation between a first position in which it is moved towards the first chamber causing a deformation of the polymeric element and the closure of the passage, and a second position in which such pusher is moved towards the second chamber so as to release the polymeric element and cause the opening of the passage.

## Description

### FIELD OF THE INVENTION

The present invention concerns the field of devices for measuring gas exchanges that occur between a plant and the surrounding environment, in particular a device for measuring gas exchanges comprising a first and a second chamber in use that are fluidically isolated from each other, in which one chamber is configured to contain the crown of the plant and the other chamber the roots of that plant.

### STATE OF THE ART

In the field of devices for measuring gas exchanges between a plant and its surrounding environment, solutions are widely available on the market that can be used to analyse the CO2 and H2O flows between soil, plants and the atmosphere. However, currently existing solutions can be used to measure small portions of the plant, e.g. individual leaves, or small portions of soil, providing local information on these exchanges. Taking measurements on the entire crown and the entire soil-root system improves the precision and ease with which a balance of the total carbon and water flows of the plant can be made, thus allowing the plant's respiration and total photosynthesis to be quantified without adopting mathematical models that receive the information detected on portions of leaves and/or soil as input to estimate the desired results described above. To solve these problems, further solutions have been developed that involve the creation of devices comprising a first and a second chamber configured to contain the crown and roots of the plant under examination respectively, and in each chamber the measurement of gas exchanges is performed. However, these solutions also have further disadvantages, such as the contamination of the gas exchanges of the root with those of the crown due to the diffusion of CO2 and H2O between the first and second chambers, since the chambers are communicating to allow the passage of the plant stem from one chamber to the other. Attempts have been made to solve this problem, e.g. the use of sealants such as glues, mastics, foams, etc., or to provide a perforated separation surface between the first and second chambers inside which the plant stem is placed. However, these solutions are time-consuming since the plant stem must grow to a size such as to be able to close the fluid communication between the first and second chambers, and at the same time they can be used in a limited time interval since the growth of the plant can cause a breakage of the sealant or the perforated surface and/or limit the normal development of the plant's conductive vessels. Other solutions instead involve the pressurization of one of the two chambers of the device, in order to be able to counteract the entry of gas from the non-pressurized chamber and perform the measurement of gas exchanges on the pressurized chamber. However, it is possible to perform one measurement at a time, in particular of the pressurized chamber only, which is also in this case a time-consuming solution.

It is therefore an ever-present need to create simple solutions from a construction point of view, which allow the first and second chambers of the device to be fluidically isolated in order to be able to perform gas exchange analyses in both chambers at the same time, and without having to destroy the plant at the end of the analyses to remove it from this device.

### SCOPE AND SUMMARY OF THE INVENTION

The present invention has the purpose of satisfying at least in part the needs indicated above, wherein this purpose is achieved by means of a device for measuring gas exchanges in a plant according to claim 1.

According to a preferred embodiment of the present invention, a device for measuring gas exchanges in a plant is presented, preferably but not limited to the flows of CO2 and H2O between soil, plant and atmosphere, through which it is possible to carry out measurements without having contamination between the gas flows exchanged between the roots and the crown of the plant and the respective environment in which such exchange of gas flows occurs. According to a further aspect of the present invention, this device has a construction configuration such as to be able to house a plant, e.g. a plant with roots buried in a pot, and carry out the desired measurements in a non-destructive manner for the plant, i.e. at the end of the measurements it is possible to remove the plant from the device without causing damage to the roots, the stem and the crown and without having to cut parts of it in order to be able to extract it from the device itself, as instead happens in traditional solutions. To achieve this result, such a device comprises a container, a closing element, e.g. a lid or a cap, releasably engaged on the container so as to fluidically isolate such container from the external environment, e.g. by providing a sealing ring. Furthermore, such a device comprises a first and a second hollow body, preferably each in the shape of a half-shell, housed in the container and rigidly connected to each other in a releasable manner defining a first chamber, in which when the device is in operation a first portion of the plant is housed, preferably the roots. In this way, when the container is closed by the closing element, the space in the container surrounding the first and the second hollow body defines a second chamber, in which when the device is in operation a second portion of the plant is housed, preferably the stem and the crown. Furthermore, in order to be able to implement this measurement setup, the first and second bodies are also shaped so as to have a first and a second recessed portion facing each other so as to define a cavity that extends from a portion of the surface on the side of the second chamber towards the first chamber. This cavity has an opening on the bottom through which it is possible to access the first chamber from the second chamber, and this opening is used to allow the stem of the plant to emerge from the first chamber. Furthermore, one of the first and second chambers can be fluidically connected at the inlet and outlet respectively to a gas delivery line and a gas evacuation line in order to define a controlled atmosphere in the device, in particular in the chamber fluidically connected to such gas delivery and evacuation lines, when the closing element is in place. To counteract any gas leakage between the first and second chamber, the measuring device comprises a fluidic seal interposed between the first and second hollow body, e.g. housed in respective perimeter grooves made on the interface surfaces to connect the first and second hollow body. To ensure fluidic isolation between the first and second chamber, since the fluidic seal is interrupted peripherally by the opening of the cavity, the measuring device also comprises an elastically deformable polymer element engaged in the cavity in a releasable manner. In particular, such a polymeric element has a longitudinal hole that defines a passage for the stem of a plant to be examined having the roots in the first chamber and the crown in the second chamber. According to the invention, the elastic deformation property of the polymeric element is exploited to interrupt the fluid communication between the first and second chambers, in particular it is deformed in use to close the longitudinal hole of the polymeric element. In particular, to achieve this result, such a device comprises a pusher engaged in the cavity on the side of the second chamber in a manner movable upon translation between a first position in which it is moved towards the first chamber causing a deformation of the polymeric element and the closure of the passage, and a second position in which such a pusher is moved towards the second chamber so as to release the polymeric element and cause the opening of the passage. For example, such a pusher can be a knob that has a threaded end engaged in a portion of the cavity, also threaded so that a rotation of the knob causes an axial movement of the same towards the first chamber which causes a compression of the polymeric element which, by deforming, closes the passage through which the stem of the plant extends, and the measurement of gas exchanges in one or both chambers can be performed without having gas contamination between one chamber and the other.

### DESCRIPTION OF THE DRAWINGS

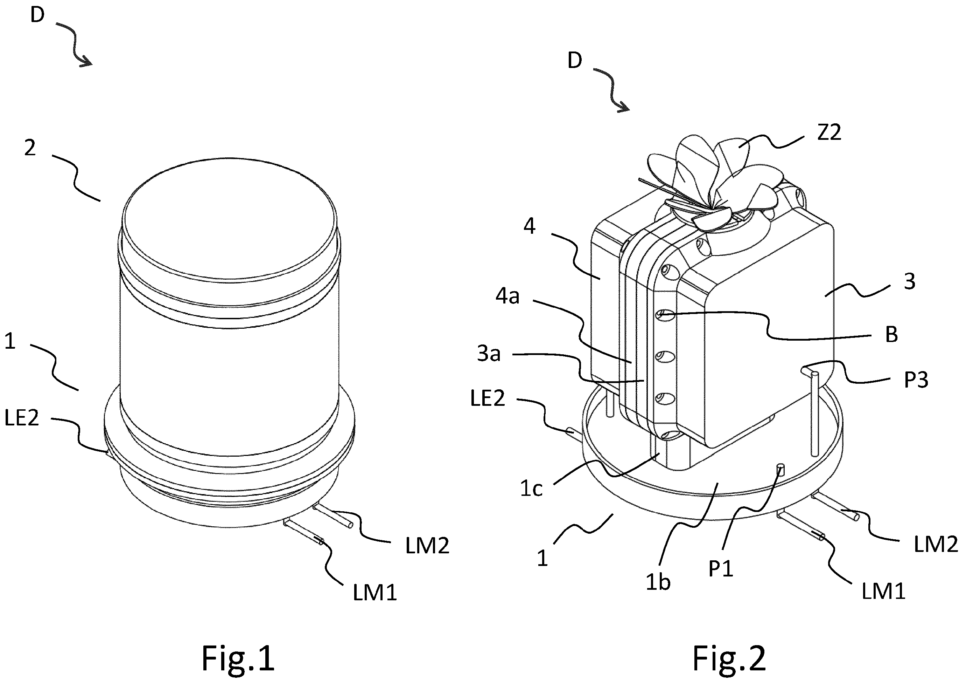

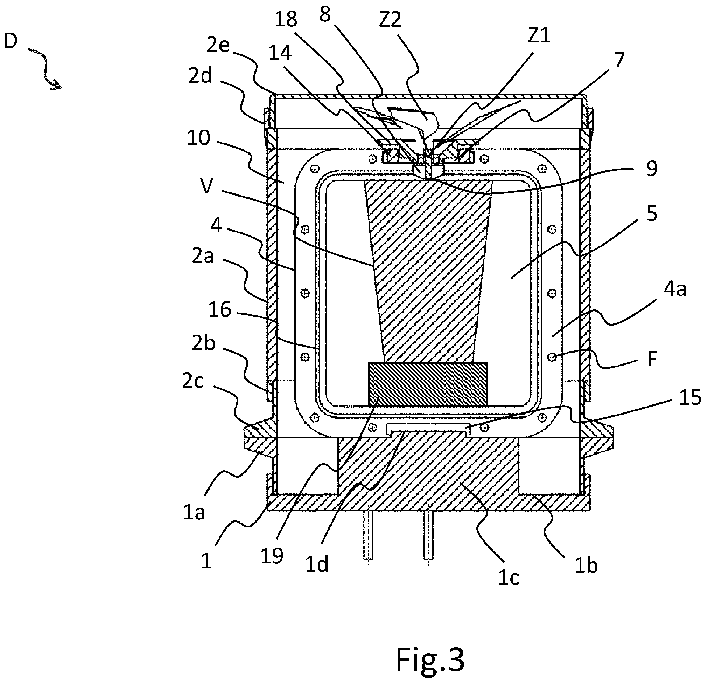

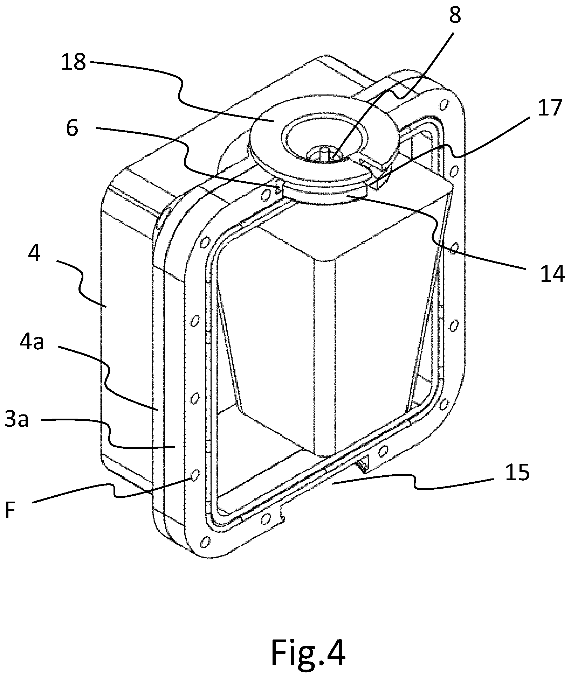

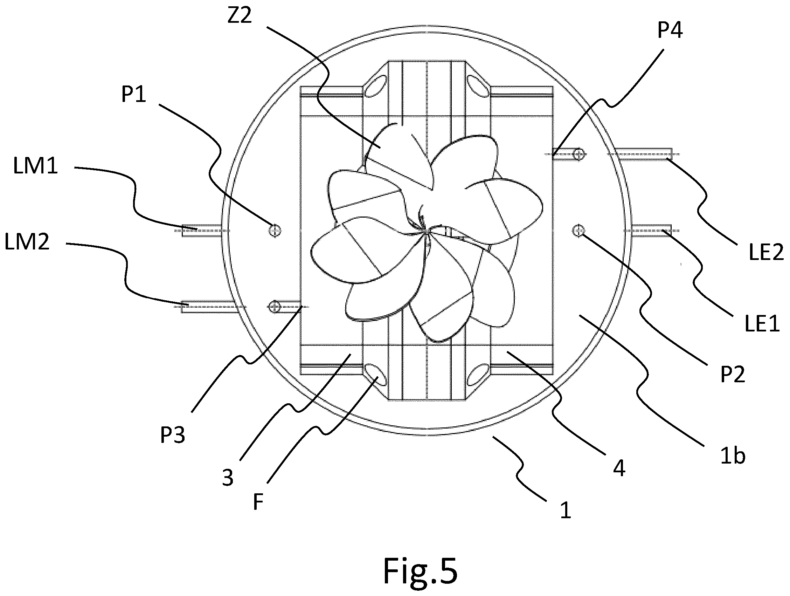

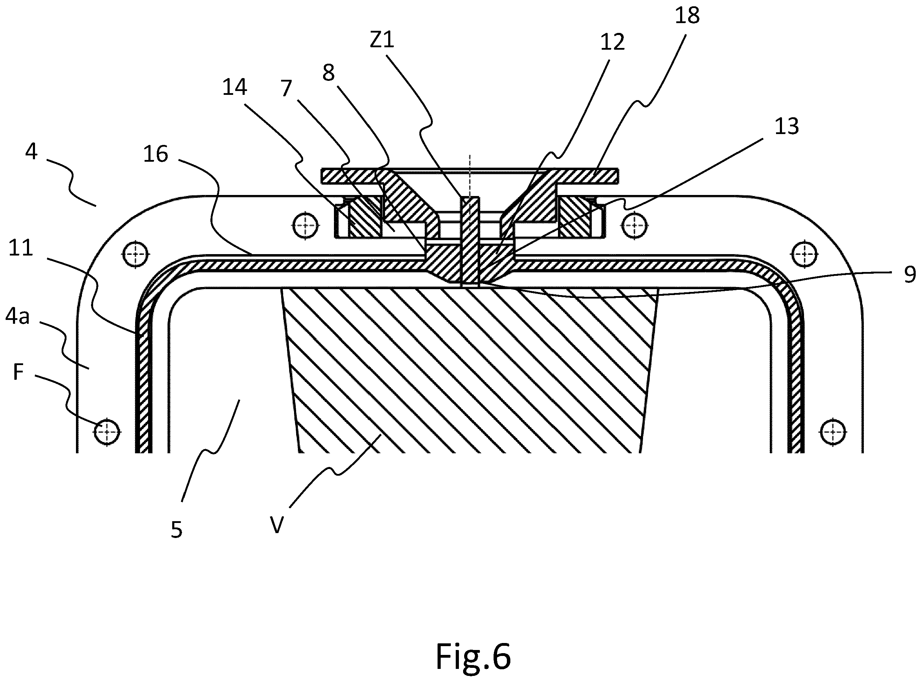

The construction and functional characteristics of the device for measuring gas exchange in a plant may be better understood from the detailed description that follows, in which reference is made to the attached figures that represent a preferred and non-limiting embodiment thereof, in which:
● Fig.1 shows a perspective view of the device for measuring gas exchange according to a preferred embodiment of the present invention;
● Fig.2 shows a perspective view of the measuring device of Fig.1 without the closing element;
● Fig.3 shows a sectional view of the measuring device of Fig.1 in an assembled configuration;
● Fig.4 shows a detailed perspective view of the first body and a portion of the second body of the device of Fig.1, and of a plant arranged in a gas exchange measurement configuration;
● Fig.5 shows a top view of the measuring device shown in Fig.2;
● Fig.6 shows a detailed sectional view of the measuring device shown in Fig.3

### DETAILED DESCRIPTION OF THE INVENTION

According to a preferred embodiment of the present invention, Fig. 1 shows a perspective view of a device D for measuring gas exchanges in a plant, in particular the flows of CO2 and H2O between soil, plant and atmosphere. To achieve this result, this device has a construction configuration such that it can house a plant, e.g. a plant with roots buried in a pot, and perform the desired measurements in a non-destructive manner for the plant, i.e. at the end of the measurements it is possible to remove the plant from the device without causing damage to the roots, stem and foliage and without having to cut parts of it in order to extract it from the device itself. In particular, this measuring device comprises a container 1 and a closing element 2, e.g. a lid preferably made of transparent material, which is used in use to close the container so that the latter is fluidically isolated from the external environment. For example, the closing element 2 may be a cap and the container 1 is open at its top, so that the cap may be mounted on that top, e.g. by interference. Preferably, as shown in Fig. 3, the closing element 2 may be formed by a plurality of pieces assembled together when the device is in operation, e.g. a central body 2a comprising a first longitudinal end 2b on which a first flanged portion 2c is mounted in use facing and releasably engaged on a second flanged portion 1a that extends from the container 1. Furthermore, the central body 2a has a second longitudinal end 2d, preferably flanged so as to define a perimeter seat on which a lid 2e is rigidly engaged, e.g. glued. Furthermore, a sealing ring (not shown in the figure) may be provided, e.g. an o-ring, mounted on the container 1 or on a perimeter edge of the closing element 2 so that when the latter is engaged on the container, the sealing ring is interposed between the container and the closing element, thus fluidically isolating the container from the external environment. Furthermore, as shown in Fig.2, the measuring device D comprises a first and a second hollow body 3, 4, e.g. a pair of preferably symmetrical shells, which are rigidly connectable to each other in a releasable manner so as to define in use a first chamber 5 (Fig.3). For example, the first and the second hollow body 3, 4 each have a respective cavity which is brought facing each other to create the connection between these bodies. Furthermore, these bodies have flanged end edges 3a, 4a which are used to rigidly connect the first and the second body, in particular they are brought into contact and rigidly constrained in a releasable manner, e.g. by means of bolts B inserted inside concentric holes F made around the perimeter of the respective flanged edges. In this way, when the first and second hollow bodies 3, 4 are connected to each other, the space between the respective cavities defines the first chamber 5. It should be noted that to measure the gas exchanges in a plant to be examined, a first portion of the plant itself is placed in use in the first chamber 5, preferably the roots, e.g. buried in a vase V. In order to achieve this result, the first and second hollow bodies 3, 4 are connected outside the container 1 so that the roots of the plant are placed in the first chamber 5 and the stem together with the crown extends outside said first chamber. For example, the first and second bodies are two half-shells that are connected so as to contain the vase V with the roots of the plant to be examined. Therefore, in order to be able to carry out gas exchange measurements once the roots of the plant are in the first chamber 5 and a section of the stem and the crown have extended out of the first chamber, it is necessary to house the plant in container 1. As can therefore be understood, the first and second hollow bodies 3, 4 are sized so that they can be housed in an assembled configuration inside container 1. Furthermore, as shown in the cross-sectional views in Fig.3-4, in order to be able to extend the stem Z1 and the crown Z2 of the plant to be examined outside the first chamber 5, the first and second bodies 3, 4 are shaped so as to have respectively a first and a second recessed portion 6, 7 preferably in an apical portion and which, in an assembled configuration, face each other and are in contact so as to define a cavity 8 that extends from a portion of the perimeter surface of each hollow body towards the first chamber 5. Furthermore, this cavity 8 is shaped so as to have an opening 9 on the side of the first chamber 5, and this opening makes the first chamber 5 accessible via the cavity 8. On the basis of this construction configuration, the cavity 8 is used so that the stem Z1 of the plant to be examined extends along the cavity outside the first chamber 5 via the opening 9. In particular, in order to achieve this result, when connecting the first and second hollow bodies 3, 4, : the stem of the plant is interposed between the first and the second recessed portion 6, 7 so that when these bodies are brought together and connected, at least one section of the stem of the plant is surrounded by the cavity 8 and its roots (not shown in the figure) are arranged in the first chamber 5, e.g. buried in a pot. Preferably, the opening 9 is defined by a first and a second 'C' shaped end profile respectively formed on the first and second recessed portion 6, 7 on the side of the first chamber 5. The stem Z1 thus extends along the cavity 8 presenting the crown Z2 with the leaves protruding out of the first and second hollow body 3, 4. In this way, as shown in Fig. 2, the plant to be examined can be housed inside the container 1 and the closing element 2 can be mounted so as to define the test setup for carrying out the measurements of the gas exchanges in the plant under examination. Preferably, the assembled first and second bodies 3, 4 may be rigidly releasably fixed to the container 1. To do this, as shown in more detail in Fig.3, the container 1 may have a bottom surface 1b from which extends a longitudinal relief 1c on which the assembled first and second bodies 3, 4 are fixed in use. For example, the perimeter surface of the first and second bodies axially opposite to that on which the cavity 8 is made is shaped so as to define in use a recess 15 shaped so as to be movably engageable in translation, e.g. by defining a shape fit, on a projecting portion 1d extending on the top of the longitudinal relief 1c transversely to the direction of extension of such relief. In this way, the projecting portion 1d defines a guide on which, for example, the assembled first and second bodies 3, 4 can slide once the recess 15 is engaged on this projecting portion, while the remaining portion of the first and second bodies 3, 4 remains supported on the surface of the longitudinal relief 1c. In particular, as shown in Fig.3, when the container 1 is closed and the plant is inside this container with the roots in the first chamber 5, the space in the container 1 around the first and second hollow bodies 3, 4 defines a second chamber 10 in which at least one section of the stem Z1 of the plant and the crown Z2 are arranged. Furthermore, to perform gas exchange measurements, one of the first and second chambers 5, 10 can be fluidically connected at the inlet to a first gas delivery line L1, e.g. through a first input port P1 made on the container 1 or on one of the first and second hollow bodies 3, 4. For example, the first gas delivery line LM1 can be a tube that can be fluidically connected to one of the first and second chambers 5, 10, inside which e.g. air is supplied at predefined conditions of temperature, humidity and CO2 concentration to define a controlled atmosphere inside the chamber to which this gas delivery line is fluidically connected. Furthermore, the chamber being measured can be connected at the output to a first gas evacuation line LE1, e.g. through a first output port P2 made on the container 1 or on one of the first and second hollow bodies 3, 4. For example, the first gas evacuation line may be a tube fluidically connectable to one of the first and second chambers 5, 10, so as to be able to extract a flow of the gas previously supplied to the chamber under examination via the first delivery line. Preferably, to perform gas exchange measurements in both the first and second chambers 5, 10, it is possible to provide a second gas delivery line LM2 and a second gas evacuation line LE2 that can be connected in a similar way to the first gas delivery and evacuation line respectively at the inlet and outlet of the chamber that does not provide the fluidic connection to the first gas delivery and evacuation line, e.g. respectively via a third and fourth port P3, P4. By way of example, this last preferred embodiment is shown in more detail in Fig.5, where the first and second gas supply lines LM1, LM2 and the first gas evacuation lines LE1 and LE2 are shown with their respective ports through which fluid communication with the first and second chambers 5, 10 is defined. It should also be noted that in order to avoid gas contamination between the first and second chambers 5, 10, i.e. between the roots and the crown Z2 of the plant under examination, it is necessary to interrupt the fluid communication between the first and second chambers. To achieve this result, a fluid seal 11 is interposed between the first and the second hollow body 3, 4. Preferably, as shown in Fig. 3, on the respective surface of the flanged edge of each hollow body 3, 4, a perimeter groove 16 is made which extends perimetrically from the respective edge defining the opening 9 so that, when the first and the second body 3, 4 are connected to each other, these grooves face each other and the fluid seal 11 (Fig. 6) is operatively housed at least in part in each of these grooves. In this way, the fluid seal 11 counteracts any leaks or gas seepage from the first to the second chamber 5, 10 and vice versa. Based on this construction configuration, however, the fluid communication is partly interrupted between the first and second chambers 5, 10, since the opening 9 still remains free to allow the passage of gas, the latter having a diameter larger than the stem Z1 of the plant to allow the latter to escape and extend out of the first chamber 5. According to the invention, as shown in Fig.6, this measuring device D comprises an elastically deformable polymeric element 12 engaged in the cavity 8 in a releasable manner so as to be able to fluidically isolate the first chamber 5 from the second chamber 10. To achieve this result, this polymeric element 12 is shaped so as to be able to be housed inside the cavity 8, e.g. when the first and second bodies 3, 4 are connected to each other, presenting a longitudinal hole 13 through which a first end faces the opening 9 and a second end axially opposite to the first on the side of the second chamber 10. In particular, this longitudinal hole defines in use a passage for the stem Z1 of the vegetable being examined through the polymeric element 12. Preferably, this polymeric element is made in two pieces each having a longitudinal groove, and each piece during the assembly of the first and second hollow bodies 3, 4 is engaged respectively on the first and second recessed portion 6, 7 so that when the first and second bodies are connected, the two pieces defining the elastomeric element 12 come into contact with the respective facing groove surrounding the stem of the vegetable being examined. Preferably, the polymeric element 12 can be a single piece, e.g. cylindrical in shape, in which a radial opening 17 is made having a transverse dimension smaller than that of the shaft Z1, e.g. a slot, which extends axially so as to make the longitudinal hole 13 accessible. In particular, the polymeric element 12 has mechanical properties such that it can be elastically deformed by hand so as to widen the radial opening 17 and thus be able to insert a section of the shaft Z1 inside the longitudinal hole 13. In this way, when the shaft Z1 is inside the longitudinal hole, the user can release the portions of the polymeric element so that the radial opening 17 returns to the size before the deformation. In this way, when the first and second hollow bodies 3, 4 are assembled, the roots are arranged in the first chamber 5 while the crown Z2 is outside this chamber. Preferably, this polymeric element 12 can be made as a single unit together with the fluid seal 11. According to a further aspect of the present invention, the elastic deformation property of the polymeric element 12 is exploited to interrupt in use the fluid communication between the first and second chambers 5, 10 through the opening 9, and in particular the polymeric element 12 is deformed so that the passage defined by the longitudinal hole 13 is closed. To do this, the measuring device D comprises a pusher 18 engaged in the cavity 8 on the side of the second chamber 10 in a movable manner to the translation between a first position in which it is moved towards the first chamber 5 causing a deformation of the polymeric element 12 and the closure of the passage, in particular of the longitudinal hole 13, and a second position in which this pusher is moved towards the second chamber 10 so as to release the polymeric element and cause the opening of the passage. For example, the first and second recessed portions 6, 7 may have a section on the side of the second chamber 10 with a diameter greater than that of the cavity 8, and within this section a threaded ring 14 is rigidly engaged. Based on this constructional configuration, the pusher 18 may be a knob having a threaded end engaged at least in part in the threaded ring 14 so that a rotation of the knob causes an axial movement of the same towards the polymeric element 12, which in turn is deformed when the end of this knob is compressed in contact with it. It should be noted that when the polymer element 12 is made as a single piece and shaped so as to define the radial opening 17, both the threaded ring 14 and the pusher 18 are in turn shaped so as to be angularly interrupted to define in use an access passage to the radial opening 17. In this case, the polymer element 12 and the threaded ring are fixed in use to one of the first and second bodies 3, 4 and positioned so that the radial opening 17 is accessible via the angular interruption of the profile of the threaded ring. The pusher 18, for example a knob, can be thus engaged with the threaded ring 14 and rotated so that the angular interruption of its profile also faces the radial opening 17 of the polymeric element 12. When this angular alignment is achieved, the stem Z1 can be inserted into the radial opening 17 and the other between the first and second body 3, 4 can be mounted so as to define the first chamber 5. Preferably, proportional valves (not shown in the figure) or any device that can vary the pressure drop on the gas evacuation lines can be applied on the respective gas discharge lines LE1, LE2. On the respective gas delivery lines LM1, LM2, on the other hand, any device that can generate a pressurized flow can be connected. Alternatively, on the gas evacuation lines, any device that can generate a suction flow, such as pumps, compressors, fans, pressurized or under-pressurized tanks, can be connected. Preferably, in order to accommodate 5 pots of different heights inside the chamber, it is possible to provide a base 19 which is fixed in a releasable manner on the bottom surface of one of the first and second bodies 3, 4, so that when these are assembled, the pot is supported on this base so that at least a section of the stem Z1 and the crown Z2 are arranged outside the cavity 8.

## Claims

1. Device (D) for measuring gas exchanges in a plant comprising:
- A container (1),
- A closing element (2) releasably engaged on the container so as to fluidically isolate such container from the external environment,
- A first and a second hollow body (3, 4) housed in the container (1) and rigidly connected to each other in a releasable manner defining a first chamber (5), and the space in the container around the first and second hollow body (3, 4) defining a second chamber (10), and said first and second body being further shaped so as to have respectively a first and a second recessed portion (6, 7) facing in use defining a cavity (8), and the first chamber (5) being accessible from the second chamber (10) via such cavity, and one of the first and second chambers being fluidically connectable at the inlet and outlet respectively to a first delivery line and a second gas evacuation line (LM1, LE1) to define a first controlled atmosphere in the device when the closing element (2) is in operation,
- A fluid seal (11) interposed between the first and the second hollow body (3, 4),
- An elastically deformable polymeric element (12) engaged in the cavity (8) in a releasable manner, and comprising a longitudinal hole (13) that defines a passage for the stem (Z1) of a plant having the roots in the first chamber and the crown (Z2) in the second chamber,
- A pusher (18) engaged in the cavity (8) on the side of the second chamber (10) in a movable manner upon translation between a first position in which it is moved towards the first chamber (5) causing a deformation of the polymeric element (12) and the closure of the passage, and a second position in which this pusher is moved towards the second chamber (10) so as to release the polymeric element and cause the opening of the passage.

2. Device (D) according to claim 1, wherein the other of the first and second chambers (5, 10) being fluidically connectable at the inlet and outlet respectively to a second delivery line and a second gas evacuation line (LM2, LE2) to define a second controlled atmosphere in the device when the closing element (2) is in operation.

3. Device (D) according to claim 1 or 2, wherein the polymeric element (12) comprises a first and a second portion each having a respective longitudinal groove, and such first and second portions being in contact when the first and second bodies (3, 4) are connected to each other so that the respective grooves are facing each other surrounding the stem (Z1) of the plant under examination.

4. Device (D) according to claim 1 or 2, wherein the polymeric element (12) is carried by one of the first and second bodies (3, 4) and has a radial opening (17) that extends axially along the direction of extension of the stem (Z1) of the plant, such opening having a transverse dimension smaller than the transverse dimension of the stem (Z1) and the polymeric element (12) being manually deformable so that when a tension is applied in a transverse direction to the opening, the radial opening widens having a transverse dimension greater than the transverse dimension of the stem making the longitudinal hole (13) accessible through such radial opening (17).

5. Device (D) according to any of the preceding claims, further comprising a threaded ring (14), and the cavity (8) having a first section with a first diameter in which the polymeric element (12) is housed and a second section on the side of the second chamber (10) having a second diameter larger than the first diameter and in which the threaded ring (14) is rigidly engaged, and the pusher (18) comprising a knob at least partly engaged in the threaded ring (14).

6. Device (D) according to claim (5), when dependent on claim (4), wherein the threaded ring (14) and the knob are each shaped so as to have a first and a second angularly interrupted profile respectively, and the radial opening (17) of the polymeric element (12) being accessible via such first and second angularly interrupted profile.

7. Device (D) according to any of the preceding claims, wherein the first and the second body (3, 4) have a first and a second flanged edge (3a, 4a) defining connection interfaces of such first and second body, and one of the first and the second flanged edge or both having a perimeter groove (16) in which the fluid seal (11) is housed.

8. Device (D) according to any of the preceding claims, wherein the fluid seal (11) and the polymeric element (12) are made of one piece.

9. Device (D) according to any of the preceding claims, wherein the container (1) has a bottom surface (1b) from which a longitudinal relief (1c) extends, and the perimeter surface of the first and second bodies (3, 4) axially opposite to that on which the cavity (8) is made is shaped so as to define in use a recess (15) shaped and releasably engaged on a projecting portion (1d) extending on the top of the longitudinal relief (1c) transversely to the direction of extension of such relief so as to rigidly lock the first and second bodies (3, 4) to the container (1).
